# EUROPÄISCHE PATENTANMELDUNG

(11) **EP 2 301 362 A1**
(43) Veröffentlichungstag der Anmeldung: **30.03.2011**
(21) Anmeldenummer: 09171506.0
(22) Anmeldetag: 28.09.2009
(51) Int. Cl.: A21D 8/04, A61K 35/74, A61P 17/10, A61P 31/10, A61K 36/899

(54) **Mischung auf Basis eines Sauerteigs**

(71) Anmelder: Woresan GmbH, 30916 Isernhagen (DE)
(72) Erfinder: Die Erfindernennung liegt noch nicht vor
(74) Vertreter: von Kreisler Selting Werner

(57) **Zusammenfassung**

Mischung erhältlich durch ein Verfahren umfassend die folgenden Schritte:
a) zu einem Roggenmehl oder Roggenschrot wird eine Kultur mit angekeimten Roggenkörnern und Wasser gegeben, die Mischung einem Erhitzungsprozess auf 30-34°C innerhalb von 3-5 Stunden ausgesetzt,
b) gefolgt von einer weiteren Zugabe von Roggenmehl oder Roggenschrot, Wasser sowie eines bakteriologischen Impfguts aus der Gruppe der heterofermentativen Milchsäurebakterien,
c) der Ansatz wird bis zur Einstellung der Stoffwechseltätigkeit der Mikroorganismen ausgesäuert und ggf. bei 90° - 95° pasteurisiert wird.
d) der Ansatz wird durch Zentrifugation in Lösung und Niederschlag getrennt, wonach die Lösung gegebenenfalls noch mindestens einmal filtriert wird.
Die Mischung kann als Arzneimittel verwendet werden, insbesondere zur Behandlung von bakteriellen, viralen Infektionen und/oder Pilzinfektionen.

## Beschreibung

Gegenstand der vorliegenden Erfindung ist eine Mischung auf Basis eines Sauerteiges, ein Arzneimittel enthaltend die erfindungsgemäße Mischung, die Verwendung der erfindungsgemäßen Mischung zur Behandlung von Erkrankungen sowie ein Verfahren zur Herstellung der Mischung.

Aus der DE-A-38 02 840 2 ist bereits ein Verfahren zur Herstellung eines Milchsäure und lebensfähige Milchsäurebakterien enthaltenden Gärproduktes durch Versäuern einer wässrigen Aufschlämmung von gebackener Brotmasse auf Sauerteigbasis in milchsaurem Medium bekannt geworden, wobei als Brotmasse das Backprodukt eines ausgesäuerten Brotteiges eingesetzt wird. Dieses Gärprodukt soll als u. a. als Hilfsmittel bei der Behandlung des ganzheitlichen Organismus von Mensch, Tier und Pflanze einsetzbar sein.

Aus der DE-A-38 46 186 ist es auch bereits bekannt geworden, aus dem Gärschlamm der Spontanversäuerung von Sauerteigbroten gewonnene Präparate z. B. als Packungszuschlagstoff zur Behandlung von chronisch entzündlichen Krankheiten der Haut einzusetzen.

Durch die DT-B-26 11 972 ist es bereits bekannt geworden, zum Herstellen eines Sauerteigs für die Bereitung von Broten und Backwaren aus Getreideschroten und/oder Mehlen in der den Vorteig bildenden Getreidemaische die Sauerteigbakterien bis zur Einstellung jeglicher bakteriologischer Stoffwechselaktivität auszusäuern.

In der EP-B-0 530 861 ist eine topische, antimikrobielle pharmazeutische Zusammensetzung offenbart, die ein Gemisch aus C₆- bis C₁₈-Fettsäuren umfasst.

DE-U-299 23 627 betrifft ein Heil- und Pflegemittel zur Förderung der Durchblutung, zur Lösung von Muskelverspannungen, zur Hautreinigung und zur Hautregenerierung, das auf Körperteile aufgetragen oder als Zusatz zu einem Wasserbad verwendet wird. Das Produkt ist **dadurch gekennzeichnet, dass** es von einem Fermentationsprodukt eines ausgesäuerten Sauerteiges gebildet ist. In dieser Druckschrift wird nichts hinsichtlich einer Vorbehandlung des als Substrat verwendeten Roggenschrots erwähnt.

WO-A-00/10395 offenbart ein Verfahren zur Herstellung eines lebenden, flüssigen Sauerteigprodukts, das nach kalter Lagerung für die Dauer von 10 Wochen mit einer Plackbildungskapazität von mindestens 5 x 10⁸ CFU/g Mehl in der Trockner aufweist. Keinerlei Hinweise werden gegeben, dass dieses Produkt als Heil- und Pflegemittel geeignet ist. In diesem Dokument wird als Puffermittel Calciumkarbonat eingesetzt, damit der pH Wert der dort beschriebenen Mischung nicht zu tief absinkt.

Die EP-A-1 458 334 betrifft Fermentationsprodukte von Roggenschrot oder Roggenmehlen mit heterofermentativen Milchsäurebakterien, die vor der Fermentation mit α-Amylase behandelt worden sind, ein Verfahren zu deren Herstellung und Anwendungen als Heil- und Pflegemittel.

Überraschenderweise wurde gefunden, dass eine neue Mischung, erhältlich durch ein Verfahren umfassend die folgenden Schritte:
a) zu einem Roggenmehl oder Roggenschrot wird eine Kultur mit angekeimten Roggenkörnern und Wasser gegeben und die Mischung einem Erhitzungsprozess auf 30-34°C innerhalb von 3-5 Stunden ausgesetzt, wobei durch enzymatische Reaktionen eine starke Maltosebildung einsetzt,
b) gefolgt von einer weiteren Zugabe von Roggenmehl oder Roggenschrot, Wasser sowie eines bakteriologischen Impfguts aus der Gruppe der heterofermentativen Milchsäurebakterien,
c) der Ansatz wird bis zur Einstellung der Stoffwechseltätigkeit der Mikroorganismen ausgesäuert und auf 90 - 95° erhitzt, bis keine Stoffwechseltätigkeit mehr nachgewiesen werden kann.
d) der Ansatz wird durch Zentrifugation in Lösung und Niederschlag getrennt, wonach die Lösung gegebenenfalls noch mindestens einmal filtriert wird,
geeignet ist als Arzneimittel eingesetzt zu werden.

Die erfindungsgemäße Mischung zeichnet sich durch einen im Vergleich zu den Fermentationsprodukten aus EP-A-1 458 334 höheren Gehalt an Tocopherolen und anderen Wirtstoffen, wie beispielsweise α-Tocotrienol, Vitamin B1, B2, B6, Folsäure sowie Pantothensäure, aus.

Die erfindungsgemäße Mischung ist herstellbar durch das erfindungsgemäß beanspruchte Verfahren, bei dem ausgehend von angekeimten Getreide mit im Folgenden näher beschriebenen Filtrations- und Rückgrat-zentrifugationsschritten eine Aufarbeitung erfolgt. Das erfindungsgemäße Verfahren umfasst die folgenden Schritte:
a) zu einem Roggenmehl oder Roggenschrot wird eine Kultur mit angekeimten Roggenkörnern und Wasser gegeben, die Mischung einem Erhitzungsprozess auf 30 - 34°C innerhalb von 3-5 Stunden ausgesetzt, wobei durch enzymatische Reaktionen eine starke Maltosebildung einsetzt,
b) gefolgt von einer weiteren Zugabe von Roggenmehl oder Roggenschrot, Wasser sowie eines bakteriologischen Impfguts aus der Gruppe der heterofermentativen Milchsäurebakterien,
c) der Ansatz wird bis zur Einstellung der Stoffwechseltätigkeit der Mikroorganismen ausgesäuert und ggf. bei 90°- 95° pasteurisiert.
d) Der Ansatz wird durch Zentrifugation in Lösung und Niederschlag getrennt, wonach die Lösung gegebenenfalls noch mindestens einmal filtriert wird.

In einer besonderen Ausführungsform des erfindungsgemäßen Verfahrens werden vorzugsweise Lactobacillus DSM 6037 und/oder Lactobacillus DSM 6129 eingesetzt.

Erfindungsgemäß kann auf die Zugabe von Konservierungsmittel verzichtet werden.

Das Fermentationsprodukt kann typischerweise zur Brechung der Gel Struktur tiefgefroren werden. Nach dem Auftauen werden die im Produkt befindlichen Kleien, insbesondere mittels einer Ultrazentrifuge abgetrennt. Danach erfolgt gegebenenfalls ein erneutes Einfrieren. Nach dem anschließenden Auftauen kann das Produkt gegebenenfalls in mehreren Schritten durch Filtration gereinigt werden, um eventuell vorhandene Trübungsstoffe zu entfernen. Es kann sich daran eine Sterilfiltration oder Sterilisierung unter Erwärmung anschließen.

Die erfindungsgemäße Mischung kann vorteilshafterweise als Medizinprodukt zum Beispiel als Arzneimittel eingesetzt werden, insbesondere in einer galenischen Zubereitung zur äußerlichen und/oder innerlichen Verabreichung.

Hier bieten sich insbesondere Lotionen, Salben, Sprays, Cremes, Gels, Tinkturen, getränkte Kompressen,- Pflaster, OP-Tücher, Binden, Verbandsmaterial, Tampons, Ampullen, Seren, Kapseln, für die Anwendungen an.

Die erfindungsgemäße Mischung kann insbesondere für ein Medizinprodukt oder Arzneimittel formuliert werden, dass zur Behandlung von bakteriellen und/oder viralen Infektionen und/oder Pilzinfektionen eingesetzt werden kann.

Beispielsweise wirkt die erfindungsgemäße Mischung gegen Onychomycosen insbesondere Tinea unguium, die durch eine Infektion mit *Trichophyton rubrum* und *Trichophyton mentagrophytes* hervorgerufen werden, ebenso wie Tinea pedis. Auch zur Behandlung von Akne auslösenden Bakterien, wie *Propionibacterium acnes* oder *Staphylococcus Epidermidis* kann die erfindungsgemäße Mischung als Arzneimittel eingesetzt werden. Ebenso hat sich die Mischung zur Behandlung von Infektionen mit *Malassezesia furfur* bewährt, die atopische, seborrhoische Ekzeme und andere Hauterkrankungen auslöst. Zu nennen ist auch die Behandlung von Infektionen mit *Candida albicans,* der Kandidose, eine Infektionskrankheit der Schleimhäute, auslöst und in Mund, Rachen, Speiseröhre, Magen, Dünn-, Dickdarm vorkommt und bei Immunschwächeerkrankungen als oportonistischer Erreger auch innere Organe befallen kann. Dabei kann sich beispielsweise bei Erkrankungen wie Krebs, Sepsis oder AIDS Lungenentzündungen ergeben. Auch zur Verhinderung von Pilzinfektionen, bei Gabe von Antibiotika oder Cortisonhaltigen Präparaten durch Pilze und Befall der Haut und Schleimhäute, kann das erfindungsgemäße Arzneimittel eingesetzt werden.

Die erfindungsgemäße Mischung enthält auch β-1.3 Glucan. Dieses ansonsten nur schwerlöslich anfallende Produkt ist hier in Wasser gelöst und dadurch für die Verarbeitung im medizinischen Bereich besonders gut einsetzbar.

Da wasserbasierte Formulierungen ohne die möglicherweise Allergie auslösenden Emulgatoren eingesetzt werden können, ist diese Art der Verarbeitung besonders in Medizinprodukten von Vorteil.

Die erfindungsgemäße Mischung kann insbesondere als Wundspray gegen Schürfwunden, Brandwunden sowie zur Linderung von Symptomen von Windpocken oder anderen die Haut in Mitleidenschaft ziehenden Erkrankungen, wie Sonnenbrand, aber auch bei offenen Blasen eingesetzt werden.

Eine weitere Anwendung erfolgt beispielsweise mittels eines Stifts, aus dem das erfindungsgemäße Medizinprodukt freigesetzt wird, zur Behandlung von Fußnagelpilz oder Fußpilz. Bei der Behandlung mit der erfindungsgemäßen Mischung wird eine schnelle Linderung des Juckreizes und eine Reduzierung der Onychomycosen schon nach wenigen Anwendungen festgestellt. Dies unterstützt die heilende Wirkung des erfindungsgemäßen Arzneimittels. Das erfindungsgemäße Arzneimittel kann insbesondere auch im Hals-Nasen-Ohrenbereich zur Behandlung von befallenen Schleimhäuten eingesetzt werden. Genannt seien beispielsweise trockenes Mundsyndrom sowie Pilzbefall in Mund, Nase und Ohr. Auch Ekzeme lassen sich mit dem erfindungsgemäßen Mittel durch topische Anwendung in ihrer Erscheinungsform lindern und heilen.

Die erfindungsgemäße Mischung lässt sich als Ekzemschutz, Hautschutz gegen Sonnenbrand, weitere Unterdrückung von Aknesymptomen, erneute Infektion durch Onychomycosen, Infektionen, die während oder nach Operationen auftreten können, Prävention von Vaginalpilzen und ähnliches einsetzen.

Das erfindungsgemäße Medizinprodukt kann auch zur Infektionsprophylaxe eingesetzt werden.

### Beispiele:

Zur Herstellung des erfindungsgemäßen Roggengels wird ein Teil Roggenmehl 1150 und einer Kultur mit 10% des Ausgangsprodukt angekeimten Roggenkörnern mit zwei Teilen Wasser vermischt versetzt. Dieser Mischung werden Fermentationsmedien mit Lactobacillus DSM 6037 sowie Lactobacillus DSM 6129 zugegeben. Diese Mischung wird für 24 bis 48 Stunden bei Temperaturen von 30 bis 34°C fermentiert. Nach dem Start der enzymatischen Reaktion wird die Startfermentation eingeleitet durch Mischen von einem Teil Roggenmehl 1150 mit zwei Teilen Wasser. Die Fermentation ist beendet, wenn sich ein konstanter pH-Wert einstellt. Die aus der Startfermentation gewonnene Mischung wird mit 60 Teilen, bezogen auf das Gesamtvolumen der Startfermentation, mit Wasser aufgefüllt. Die Fermentationszeit beträgt weitere 24 bis 48 Stunden bis ein konstanter pH-Wert erreicht wird bei einer typischen Temperatur von 30 bis 34 °C. Danach erfolgt eine thermische Behandlung für 3 bis 5 Stunden bei einer Temperatur von 92 bis 95°C. Es werden keine Konservierungsmittel zugefügt. Die Haltbarkeit wird durch Heißabfüllung in Sterilen Behältern gewährleistet.

Produktionsschritt schließen sich die Trennverfahren zur Herstellung der Mischung an.

Das Fermentationsprodukt wird zur Brechung der Gel Struktur tiefgefroren. Nach dem Auftauen werden die im Produkt befindlichen Kleien, durch eine Ultrazentrifuge abgetrennt. Danach erfolgt ein erneutes Einfrieren. Nach dem erneuten Auftauen wird das Produkt in mehreren Schritten in Falten und Kerzenfiltern von Trübstoffen gereinigt und im Anschluss per Erhitzung bei 60° steril filtriert.

### Ausgewählte Parameter

| **Untersuchungsparameter** | | **gemäß Erfindung** | **gemäß EP-A-1458334** |
|---|---|---|---|
| Gehalt Tocopherol | | 30 µg / 100ml | 1,4 µg /1 00ml |
| | α-Tocopherol | 27 µg / 100 ml | 1,4 µg / 100 ml |
| | β-Tocopherol | < 10 µg / 100 ml | < 1 µg / 100 ml |
| | γ-Tocopherol | 10 µg / 100 ml | < 1 µg / 100 ml |
| | δ-Tocopherol | < 10 µg / 100 ml | < 1 µg / 100 ml |
| | α-Tocopherol | < 10 µg / 100 ml | < 1 µg / 100 ml |
| Gehalt Vitamin B₁ | | 9,2 µg / 100 ml | Ergebnis folgt |
| Gehalt Vitamin B₂ | | 7,9 µg / 100 ml | 1,9 µg / 100 ml |
| Gehalt Vitamin B₆ | | 21,0 µg / 100ml | 24 µg / 100ml |
| Gehalt Folsäure | | 2,6 µg / 100 ml | 0,2 µg / 100ml |
| Gehalt Pantothensäure | | 330 µg /100 ml | 83 µg / 100 ml |
| Gehalt Nicotinsäureamid | | 73 µg / 100 ml | 72 µg / 100 ml |

## Patentansprüche

1. Mischung erhältlich durch ein Verfahren umfassend die folgenden Schritte:
a) zu einem Roggenmehl oder Roggenschrot wird eine Kultur mit angekeimten Roggenkörnern und Wasser gegeben, die Mischung einem Erhitzungsprozess auf 30-34°C innerhalb von 3-5 Stunden ausgesetzt, wobei durch enzymatische Reaktionen eine starke Maltosebildung einsetzt,
b) gefolgt von einer weiteren Zugabe von Roggenmehl oder Roggenschrot, Wasser sowie eines bakteriologischen Impfguts aus der Gruppe der heterofermentativen Milchsäurebakterien,
c) der Ansatz wird bis zur Einstellung der Stoffwechseltätigkeit der Mikroorganismen ausgesäuert und bei ca. 90°- 95° pasteurisiert.
d) der Ansatz wird durch Zentrifugation in Lösung und Niederschlag getrennt, wonach die Lösung gegebenenfalls noch mindestens einmal filtriert wird.

2. Mischung nach Anspruch 1 mit einem Gehalt an α, β, γ, δ Tocopherol, α Tocotrienol; Vitamin B₁, B₂, B₆, Folsäure, Pantothensäure, Nicotinsäureamid.

3. Mischung nach Anspruch 1 oder 2 enthaltend β-1.3 Glucan

4. Arzneimittel enthaltend eine Mischung gemäß Anspruch 1 bis 3.

5. Arzneimittel gemäß Anspruch 4 in einer galenischen Zubereitung zur äußerlichen und/oder innerlichen Verabreichung.

6. Arzneimittel nach Anspruch 4 oder 5 in Form von Lotionen, Salben, Sprays, Cremes, Gels, Tinkturen, getränkte Kompressen,- Pflaster, OP-Tücher, Binden, Verbandsmaterial, Tampons, Ampullen, Seren, Kapseln, für die Anwendungen.

7. Mischung gemäß Anspruch 1 bis 3 zur Behandlung von bakteriellen, viralen Infektionen und/oder Pilzinfektionen.

8. Mischung gemäß Anspruch 1 zur Behandlung von Verletzungen, Verbrennungen der Haut und Schleimhaut, Hauterkrankungen.

9. Verfahren zur Herstellung einer Mischung gemäß Anspruch 1 oder 2 mit den folgenden Schritten:
a) zu einem Roggenmehl oder Roggenschrot wird eine Kultur mit angekeimten Roggenkörnern und Wasser gegeben, die Mischung einem Erhitzungsprozess auf 30-34°C innerhalb von 3-5 Stunden ausgesetzt,
b) gefolgt von einer weiteren Zugabe von Roggenmehl oder Roggenschrot, Wasser sowie eines bakteriologischen Impfguts aus der Gruppe der heterofermentativen Milchsäurebakterien,
c) der Ansatz wird bis zur Einstellung der Stoffwechseltätigkeit der Mikroorganismen ausgesäuert und ggf. bei ca. 90°- 95° pasteurisiert.
d) der Ansatz wird durch Zentrifugation in Lösung und Niederschlag getrennt, wonach die Lösung gegebenenfalls noch mindestens einmal filtriert wird.

10. Verfahren nach Anspruch 9, wobei die heterofermentativen Milchsäurebakterien Lactobacillus DSM 6037 und/oder Lactobacillus DSM 6129 sind.
